# EUROPÄISCHE PATENTANMELDUNG

(11) **EP 0 919 623 A2**
(43) Veröffentlichungstag der Anmeldung: **02.06.1999**
(21) Anmeldenummer: 98121736.7
(22) Anmeldetag: 14.11.1998
(51) Int. Cl.: C12N 15/70, C12N 15/73, C12N 1/04

(54) **Verfahren und Kit zur Klonierung von Fragmenten**

(30) Priorität: 17.11.1997 EP 97120089
(71) Anmelder: Roche Diagnostics GmbH, 68298 Mannheim (DE)
(72) Erfinder: Metzler, Thomas, Dr., 80337 München (DE); Sobek, Harald, Dr., 82377 Penzberg (DE); Reichhuber, Rolf, 82377 Penzberg (DE)

(57) **Zusammenfassung**

Gegenstand der Erfindung sind drei Cosmidvektoren, welche zur Klonierung von Fragmenten mit einer Größe zwischen 7 und 36 kb geeignet sind. Diese Vektoren besitzen ein E.coli ColE1 Replikon, ein Ampicillinresistenzgen, eine multiple Klonierungskassette, Cos-Sites zur *in vitro* Verpackung mit Hilfe von Lambda Packaging Extkakten sowie Fragmente aus dem Genom des Bakteriophagen Lambda. Die Lambda Sequenzen wurden so ausgewählt, daß keine lytischen Prozesse, keine Vektorinstabilitäten (Deletionen) und keine unerwünschten Rekombinationsereignisse zwischen Lambda DNA und zu dem zu klonierendem Fragment auftreten. Abhängig von der Länge des in den jeweiligen Vektor inserierten Lambda Fragments können heterologe Fragmente unterschledlicher Länge kloniert werden.

SEQUENZPROTOKOLL

(1) ALLGEMEINE ANGABEN:

(i) ANMELDER:
(A) NAME: Boehringer Mannheim GmbH
(B) STRASSE: Sandhoferstr. 116
(C) ORT: Mannheim
(E) LAND: DE
(F) POSTLEITZAHL: 68305
(G) TELEFON: 06217591456
(H) TELEFAX: 06217594457

(ii) BEZEICHNUNG DER ERFINDUNG: Verfahren und Kit zur Klonierung
von Fragmenten

(iii) ANZAHL DER SEQUENZEN: 3

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:
(A) LÄNGE: 129 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 1:

CAGGAAACAG CTATGACTAA TACGACTCAC TATAGGGAGA GCGGCCGCAT TTAAATGGCC
60

ATATAGGCCC CCGGGACTAG TGCGGCCGCG TATTCTATAG TGTCACCTAA ATACTGGCCG
120

TCGTTTTAC
129

(2) ANGABEN ZU SEQ ID NO: 2:

(i) SEQUENZKENNZEICHEN:
(A) LÄNGE: 24 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 2:

CACAAGGGCT ACTGGTTGCC GATT
24

(2) ANGABEN ZU SEQ ID NO: 3:

(i) SEQUENZKENNZEICHEN:
(A) LÄNGE: 22 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 3:

AGCTTCCCAA CGTGATCGCC TT
22

## Beschreibung

Die Erfindung betrifft ein Verfahren und einen Kit zur Klonierung von DNA Fragmenten, beispielsweise PCR Amplifikationsprodukten, die aufgrund ihrer Größe nicht mit Hilfe von Plasmiden klonierbar sind, die aber gleichzeitig zu klein sind, um in Cosmiden kloniert werden zu können. Zu diesem Zweck wurden modifizierte Cosmid-Vektoren konstruiert, welche zur Klonierung von DNA Amplifikationsprodukten und DNA-Fragmenten mit einer entsprechenden Größe geeignet sind.

Nach dem Stand der Technik sind verschiedene Methoden zur Klonierung von heterologer DNA bekannt. Die einfachste Methode ist die Verwendung von Plasmidvektoren, welche einen geeigneten Replikationsursprung, ein Antibiotikaresistenzgen als Selektionsmarker sowie mindestens eine geeignete Restriktionsschnittstelle enthalten. Werden heterologe DNA Fragmente. deren Größe 10 kb übersteigt, jedoch in herkömmliche Plasmide wie pUC oder anderen pBR-Derivaten inseriert, so führt dies erfahrungsgemäß dazu, daß solche Konstrukte selbst in rekombinationsdefizienten Wirtszellen nicht ohne Deletionen klonierbar sind. Der Nachteil dieser Methode besteht also darin, däß nur DNA Fragmente mit einer begrenzten Größe von maximal etwa 10 kb effizient kloniert werden können.

Die Klonierung größerer Fragmente erfolgt nach dem Stand der Technik mit Hilfe von Vektoren, basierend auf dem Vermehrungszyklus des Bakteriophagen Lambda. Dabei wird zunächst die in linearer Form existierende Lambda DNA präpariert. Dann wird mithilfe eines geeigneten Restriktionsenzymverdaus ein internes Fragment, der sogenannte Stuffer, entfernt. Die zu kloniernde DNA wird durch einen Ligationsschritt anstelle des Stuffers zwischen die Vektorarme inseriert (z.B. Lambda-gt10; Lambda-gt11). Anschließend wird die auf diese Weise behandelte DNA mit einem vorher präparierten Extrakt von Phagenpartikeln inkubiert.

Durch diese sogenannte *in vitro* packaging Reaktion entstehen infektiöse Phagenpartikel, mit denen geeignete Wirtsbakterien infiziert werden können. Auf diese Weise lassen sich Fragmente bis ca 20 kb klonieren (Winnacker, E.-L., In: From Genes to Clones, VCH Weinheim (1987) Winnacker, E.-L (ed), pp 154-167); Rimm, D.L. et al. (1980), Gene 12, 301-309).

Der Nachteil der Methode besteht darin, daß die auf diese Weise klonierte DNA stets im Genom des Lambda-Phagen inseriert bleibt. Das Lambda-Genom selbst enthält Gene, die für lytische Funktionen kodieren und somit zu einer Lyse der Bakterienzellen führen, so daß aufgrund einer entsprechenden Infektion stets sogenannte Plaques ausgebildet werden. Im Vergleich zur Vermehrung und Präparation von Plasmiden ist eine Vermehrung bzw. Präparation von Lambda DNA deshalb wesentlich zeit- und kosten-intensiver. Aus diesem Grunde werden Lambda-Vektoren in der Praxis auschließlich zur Anlage von Genbanken, aber nicht zur Klonierung spezieller DNA Fragmente verwendet.

Bekannt sind weiterhin sogenannte Cosmid-Vektoren, mit denen besonders große DNA Fragmente kloniert werden können. Dabei handelt es sich um Plasmide, welche zusätzlich eine sogenannte Cos Region besitzen. Diese Cos Region entstammt den terminalen Sequenzen des Genoms des Bakteriophagen Lambda und besitzt eine essentielle Funktion bei dessen Vermehrungszyklus: Bei der Replikation der Phagen DNA kommt es zur Bildung von Konkatameren. Dabei handelt es sich um Multimere des Phagengenoms, die in Tandemanordnung vorliegen. Während der Verpackungsreaktion werden die Konkatamere an den Cos-Regionen durch das A-Protein des Lambda Phagen gespalten. Dadurch entstehen dann einzelne lineare Phagengenome mit cohäsiven Enden, die anschließend in Phagenpartikel verpackt werden.

Die zu klonierenden DNA Fragmente werden zusammen mit den linearisierten Cosmid-Vektoren unter Bedingungen ligiert, bei denen *in vitro* multimere Konkatamere entstehen. Aus diesen Konkatameren entstehen dann durch Inkubation mit einem Packaging Extrakt monomere Moleküle, welche jeweils das zu klonierende Fragment zwischen 2 Cos Regionen enthalten und folglich in die Phagenpartikel des Extraktres verpackt werden. Anschließend wird eine Infektion (auch als Transduktion bezeichnet) in geeignete Wirtsbakterien durchgeführt. Im Wirt werden schließlich durch die kohesiven Enden (entstanden durch die beschriebene Spaltung der Cos Regionen durch das Protein A) der terminalen Cos Sequenzen zirkuläre Cosmide gebildet, die das zur klonierende Fragment enthalten und aufgrund ihrer Plasmideigenschaften auf einfache Art und Weise vermehrt und präpariert werden können.

Gängige Cosmide haben eine Größe zwischen 4 und 6 kb, da für Genbanken möglichst große Fragmente kloniert werden müssen (Maniatis et. al. Molecular Cloning. A Laboratoy Manual, Cold Spring Harbour, Laboratory Press, Cold Spring Harbour, NY, 1982, S. 45). Die Größe der zu klonierenden Fragmente richtet sich nach der Größe des verwendeten Cosmids: mithilfe des Lambda Packaging Systems werden DNA-Moleküle mit einer Länge zwischen 38 kb und 52 kb verpackt. Demzufolge können Fragmente mit einer Größe zwischen 38 kb und 52 kb abzüglich der Größe des verwendeten Cosmids mit hoher Effizienz kloniert werden. Wenn das Cosmid wie z.B pHC79 (Hohn and Collins (1980), Gene **11,** S. 291-298) eine Größe von 6,5 kb hat, können daher Fragmente von 31,5-45,5 kb kloniert werden.

Der Nachteil der Cosmide besteht also darin, daß nur DNA Fragmente mit einer bestimmten Größe inseriert werden können. Demnach existiert gemäß dem Stand der Technik keine Methode, welche auf einfache Art und Weise die Klonierung von Fragmenten ermöglicht, die einerseits zu klein für eine Verpackung von Cosmidvektoren sind, andererseits aber für eine Klonierung mit Hilfe von Plasmiden (ohne Cos Regionen zur *in vitro* Verpackung) zu groß sind.

Fragmente dieser Größenklasse entstehen durch PCR-Amplifkationsreaktionen, beispielsweise mit dem Expand™ Long Template PCR System (Boehringer Mannheim Katalog Nr. 1681834). Dieser Kit enthält Reagezien für ein PCR Verfahren, mit dem genomische Fragmente bis zu einer Länge von ca. 30 kb amplifiziert werden können. Eine direkte Klonierung entsprechender Amplifkationsprodukte war mit den aus dem Stand der Technik bekannten Plasmid- bzw. Cosmidvektoren bis zum Zeitpunkt der Erfindung jedoch nicht möglich. Wurde man beispielsweise versuchen, ein 27 kb PCR Produkt in ein 6,5 kb großes Cosmid zu klonieren, so wurde ein 33,5 kb großes Molekül entstehen; dies wäre jedoch für eine Lambda-Verpackung zu klein.

Gegenstand der Erfindung sind daher Cosmidvektoren, die mindestens 15 kb, aber höchstens 35 kb groß sind. Vorzugsweise besitzen die erfindungsgemäßen Cosmidvektoren Größen zwischen 15 kb und 32,6 kb. Solche Vektoren sind zur Klonierung von DNA Fragmenten mit einer Größe zwischen 5 kb und 40 kb geeignet. Insbesondere sind solche Vektoren zur effizienten Klonierung von DNA Fragmenten init einer Größe zwischen 5 kb und 37 kb geeignet.

Die Vektoren enthalten notwendigerweise Cos Sequenzen des Bakteriophagen Lambda, einen prokaryontischen Replikationsursprung, vorzugsweise ein ColE1 Replikon und ein Gen zur Selektion in antibiotikahaltigem Nährmedium, vorzugsweise ein Ampicillinresistenzgen. Zusätzlich können diese Vektoren eine Klonierungskasssette enthalten. Diese Kassette enthält eine oder mehrere Schnittstellen für beliebige Restrikitionsenzyme, sowie fakultativ flankierende Sequenzen, die als Bindestellen für universelle Sequenzierungsprimer dienen können. Zusätzlich können diese flankierenden Sequenzen Bakteriophagenpromotoren, wie SP6-, T7- oder T3-Promotoren enthalten, die zur *in vitro* Transkription des zu klonierenden Fragmentes dienen. Gegenstand der Erfindung ist daher insbesondere ein Cosmid der genannten Größenordnung, von dem ein Teil identisch mit der vollständigen Sequenz von pHC 79 ist.

Erfindungsgemäß werden die zur Konstruktion eines Cosmids mit einer Größe zwischen 15 kb und 35 kb verwendeten Sequenzen nicht beliebig ausgewählt. Werden normalerweise heterologe DNA Fragmente in Plasmide dieser Größenordnung inseriert, so führt dies häufig zu Deletionsereignissen und/oder Rekombinationsereignissen zwischen der Vektor-DNA - insbesondere zwischen der im Plasmid/Cosmid einklonierten Abstandshalter DNA - und dem zu inserierenden Fragment, sodaß eine Klonierung unmöglich oder sehr ineffizient wird.

Eine Ausführungsform für die Klonierung von DNA Fragmenten eukaryontischen Ursprungs sind Cosmide mit Abstandshaltern, deren Sequenz nicht eukaryontischen Ursprungs ist. Eine bevorzugte Ausführungsform der neuartigen Vektoren sind solche Cosmide, die außer den Cos Sequenzen als Abstandshalter ein zusätzliches DNA Fragment aus dem Genom des Bakteriophagen Lambda enthalten. Dies kann beispielsweise für Strukturproteine des Bakteriophagen kodieren. Da diese Cosmide erfindungsgemäß auch zur Klonierung von PCR-Fragmenten geeignet sind, wie sie mithilfe des Expand™ Long Template PCR Systems erzeugt werden können, werden diese Cosmide im Folgenden als Expand-Vektoren bezeichnet.

Eine besonders bevorzugte Ausführungsform sind Cosmidvektoren, bei denen zusätzlich enthaltene DNA Fragment entweder aus dem 8,5 kb großen BamHI/BclI-Fragment (Expand-Vektor III), dem 16,8 kb großem BamHI- Fragment (Expand-VektorII) oder dem 25,9 kb großen BbrI/BspLU1I Fragment (Expand-VektorI) des Bakteriophagen Lambda cI857Sam7besteht (Weigle, J.et al. (1966); Proc. Nat. Acad. Sci., U.S.A. **55**, 1462-1466). Überraschenderweise führt die Präsenz derartiger Lambda Sequenzen im Cosmid nicht zu Interaktionen mit dem Packaging System während des *in vitro* Packaging. Lytische Lambda-Funktionen sind dabei ebenfalls komplett ausgeschaltet.

Ebenfalls Gegenstand der Erfindung sind Escherichia coli Wirtszellen, die einen der beschriebenen Vektoren enthalten und somit zu dessen Replikation geeignet sind.

Gegenstand der Erfindung ist auch die Verwendung der beschriebenen Cosmidvektoren zur Klonierung von DNA Fragmenten mit einer Größe zwischen 5 kb und 40 kb, vorzugsweise von Fragmenten mit einer Größe zwischen 7 kb und 36 kb und insbesondere mit einer Größe zwischen 16,5 kb und 25 kb. Beispielsweise handelt es sich bei zu klonierenden Fragmenten dieser Größenklassen um PCR Amplifikationsprodukte. Diese können vorzugsweise mit dem Expand™ PCR System (Boehringer Mannheim, Katalog No 1681834; 681842; 1759060; 1811002) hergestellt werden. Der zu klonierende Größenbereich an DNA Fragmenten hängt dabei direkt von der Größe des erfindungsgemäßen Cosmids ab. Die folgende Tabelle 1 enthält eine Übersicht der Klonierungskapazität von drei erfindungsgemäß konstruierten Vektoren. Die drei E.coli Stämme BMTU 7337 (DSM 11839), BMTU 7338 (DSM 11840) und BMTU 7339 (DSM 11841), welche die Expand Vektoren I, II, und III enthalten, sind bei der Deutschen Sammlung von Mikroorganismen und Zellkulturen GmbH (DSM), Marschroder Weg 1b, 38124 Braunschweig, hinterlegt worden.

**Tabelle 1**

| **Expand Vektor Daten** | | | |
|---|---|---|---|
| | Größe | Abstandshalter | Klonierungskapazität |
| Vektor I | 32,6 kb | 25,9 kb | 7,0 - 16,5 kb |
| Vektor II | 23,5 kb | 16,8 kb | 16,5 - 25,0 kb |
| Vektor III | 15,2 kb | 8,5 kb | 25,0 - 36,0 kb |

Ebenfalls Gegenstand der Erfindung sind Verfahren zur Klonierung von DNA Fragmenten in Cosmidvektoren mit den bereits offenbarten Merkmalen, bei denen Fragmente mit einer Größe zwischen 5 kb und 40 kb, vorzugsweise mit einer Größe zwischen 7 kb und 36 kb und insbesondere mit einer Größe zwischen 16,5 kb und 25 kb kloniert werden. In einer bevorzugten Ausführungsform handelt es dabei um PCR Amplifikationsprodukte wie sie mit dem Expand™ PCR System (Boehringer Mannheim) hergestellt werden können.

Gegenstand der Erfindung ist ferner ein Verfahren zur Konservierung einer Escherichia coli Suspension in einer 5-20 mM MgSO₄-Lösung, vorzugsweise einer Suspension des Stammes DH5alpha, bei dem die Suspension von einer Temperatur, die niedriger als 30°C, aber höher als 4°C ist, über einen Zeitraum von mehr als 30 Minuten auf -70°C abgekühlt wird. Vorzugsweise wird die Suspension von Raumtemperatur über einen Zeitraum von mehr als 30 Minuten auf -70°C abgekühlt. Auf diese Weise behandelte Bakterien können ohne weitere Vorbehandlung als Wirtsorganismen zur Replikation von *in vitro* verpackter Cosmid-DNA verwendet werden.

Gegenstand der Erfindung ist außerdem ein Kit zur Klonierung heterologer DNA , welcher mindestens einen der beschriebenen Cosmid-Vektoren enthält. Dabei kann der Vektor in linearisierter und dephosphorylierter Form vorliegen. Zusätzlich kann der Kit ein oder mehrere Enzyme wie DNA Ligase, Polynukleotid-Kinase, oder T4 DNA Polymerase enthalten. Als weitere fakultative Komponenten kann der Kit einen Lambda *in vitro* DNA Packaging Extrakt und eine Suspension von E. coli DH5alpha in 5-20 mM MgSO₄-Lösung enthalten.

### Konstruktion der Expand Vektoren I, II und III:

### Expand Vektor I

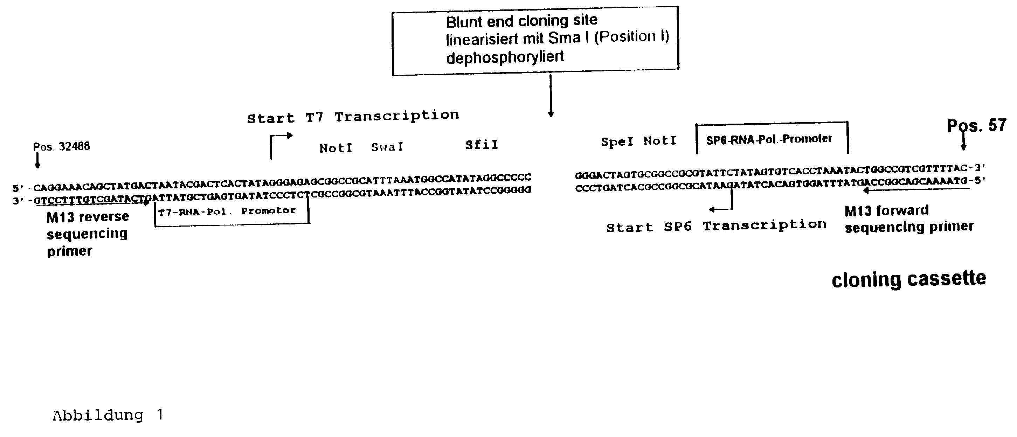

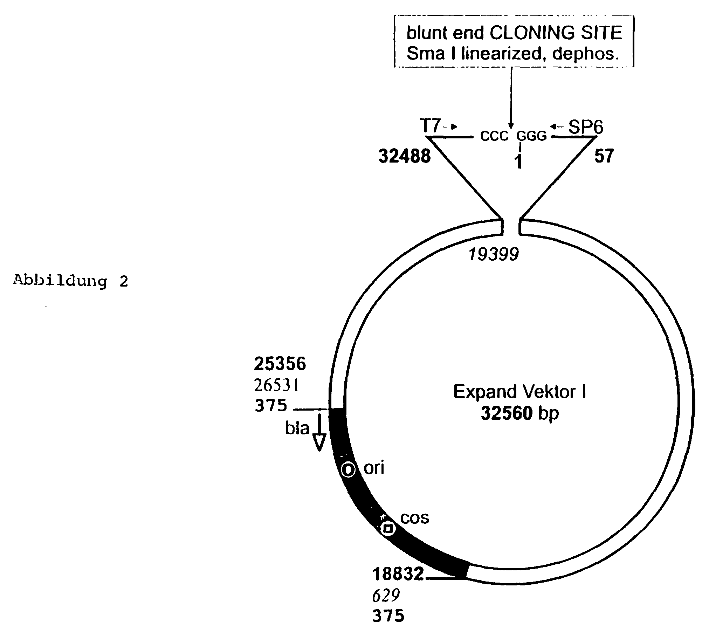

Das Cosmid pHC 79 wurde mit Bam HI linearisiert. Die hierbei entstehenden 5' überhängenden Enden wurden mit T4 DNA Polymerase aufgefüllt. Zur Vergrößerung des Cosmidvektors wurde ein DNA Abschnitt aus dem Genom des Bakteriophagen Lambda cI 857 Sam7 als Abstandshalter-Fragment in das linearisierte, blunt end Cosmid kloniert. Die Lambda DNA wurde hierzu mit den Restriktionsenzymen Bbr I und Bsp LU11 I gespalten. Das hierbei entstandene 25903 bp große Fragment wurde durch Agarose Gelelektrophorese von den übrigen Fragmenten abgetrennt und aus dem Gel isoliert. Die durch obige Spaltung erzeugten überhängenden Enden wurden mit T4 DNA Polymerase aufgefüllt. Das so erhaltene Lambda DNA-Fragment (Abstandshalter) wurde in die aufgefüllte BamHI-Schnittstelle des oben beschriebenen Cosmids ligiert. Nach Transformation in den Bakterienstamm E. coli XL I blue wurde die Cosmid DNA isoliert und mit Sma I linearisiert. In dieses Cosmid wurde eine Klonierungskassette (Abbildung 1, SEQ. ID. NO. 1) mit einer Länge von 129 Basenpaaren (durch Ligation aus 4 synthetischen Oligonukleotiden erhalten) ligiert. Diese enthält Restriktionsenzym-Schnittstellen für die Rare-Cutter-Restriktionsenzyme NotI, SwaI, SfiI und SpeI, Sequenzierprimer-Sequenzen sowie Sp6/T7 Promotoren (Abbildung 2).
Die SmaI Schnittstelle (Position 1) in der Mitte der Klonierungskassette ist singulär auf den Expand Vektoren vorhanden, da die zur Klonierung der Kassette in das Ausgangsplasmid verwendeten Sma I-Schnittstellen nicht regeneriert werden. Daher werden die zu klonierenden Fragmente in diese SmaI Schnittstelle inseriert.

Durch diese Konstruktion besitzt der Expand-Vektor I eine Größe von 32,5 Kb, einen Lambda Abstandshalter von 25,9 Kb und eine Klonierungskapazität (für das Lambda Packaging System) von 7,0 - 16,5 Kb. Dies ergibt in der Summe 39.5-49 kb und entspricht dem Größenbereich, welcher im Lambda Packaging System verpackbar ist (Abbildung 2).

### Expand Vektoren II und III

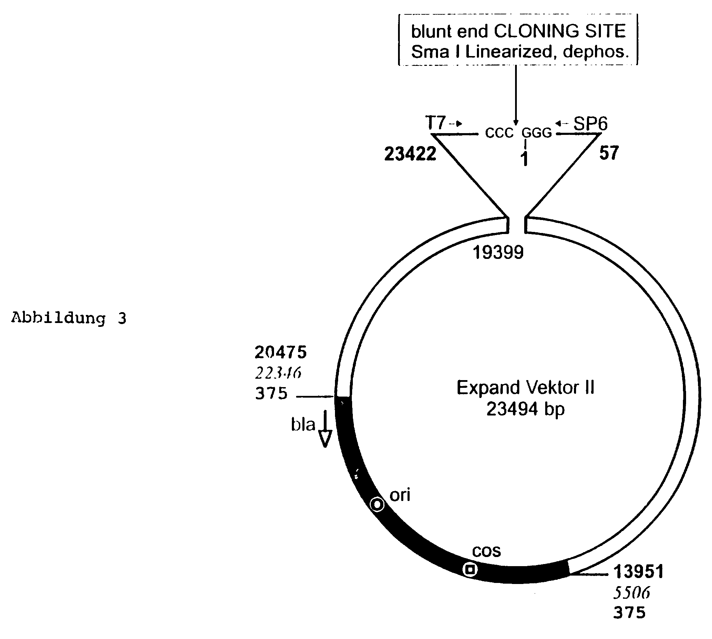

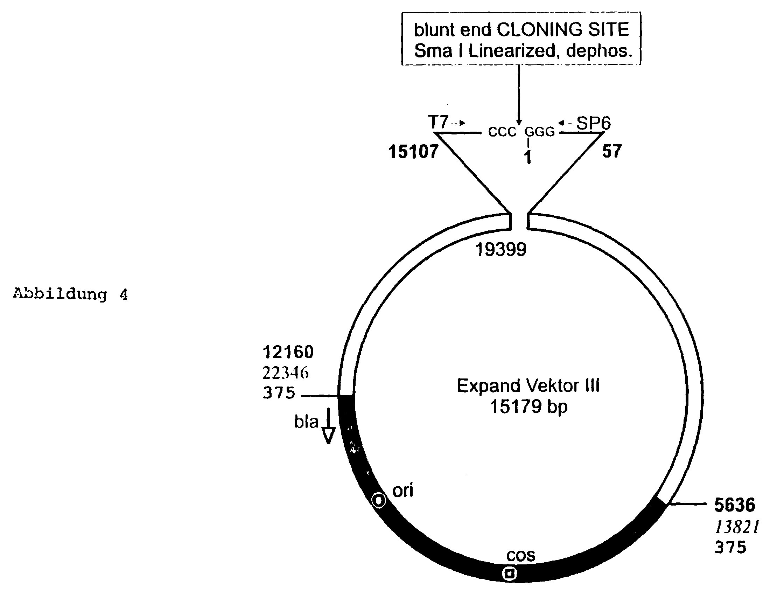

Die Konstruktion der Expand Vektoren II und III erfolgte im Prinzip analog zu Expand Vektor I. Im Falle von Expand Vektor II wurde jedoch ein 16841 bp großes BamHI Fragment aus Lambda isoliert und direkt in die Bam HI-Schnittstelle des Cosmids pHC 79 inseriert. Im Falle von Expand Vektor III wurde DNA des Bakeriophagen Lambda Lambda cI 857 Sam7 mit Bam HI/Bcl I gespalten. Anschließend wurde, ein 8526 bp großes Fragment isoliert und direkt in die Bam HI-Schnittstelle des Cosmids pHC 79 inseriert. Die Insertion der Klonierungskassette in die Expand Vektoren II und III wurde exakt wie bei der Konstruktion von Expand Vektor I durchgeführt (Abbildungen 3, 4). Durch die Insertion der Lambda Abstandshalter in pHC 79 sind Cosmid-Vektoren mit einer Größe von 23,5 kb und 15,2 kb entstanden. Diese besitzen eine Klonierungskapazität von 16,5 kb - 25 kb, bzw. 25-36 kb und können mithilfe eines Lambda Packaging Extrtaktes verpackt werden.

Somit ermöglicht die Verwendung eines der Expandvektoren I, II, oder III die Klonierung von Fragmenten in einer Größenordnung zwischen 7 und 36 kb. Dadurch können DNA- Fragmente einer bestimmten Größenklasse kloniert werden, die bisher einerseits zu groß für eine Klonierung in herkömmliche Plasmide und andererseits aber zu klein für eine Klonierung mit Hilfe von Cosmiden waren.

### Kurze Beschreibung der Abbildungen

- Abbildung 1:: Klonierungskassette, die in die Expand Vektoren inseriert wurde. Zu klonierende Fragmente werden in die singuläre SmaI Schnittstelle inseriert (SEQ. ID. No. 1).
- Abbildung 2:: Expand Vektor I, 32,6 kb, zur Klonierung von Fragmenten zwischen 7,0 und 16,5 kb
- Abbildung 3:: Expand Vektor II, 23,5 kb, zur Klonierung von Fragmenten zwischen 16,5 und 25,0 kb
- Abbildung 4:: Expand Vektor III, 15;2 kb, zur Klonierung von Fragmenten zwischen 25,0 und 36,0 kb
- Abbildung 2-4:: Fettgedrückte Zahlen entsprechen den Koordinaten der Expand-Vektoren, kursiv gedruckte Zahlen entsprechen den Koordinaten des Lambda-Genoms, alle weiteren Zahlen entsprechen den Koordinaten des pHC79 Cosmids.

Folgende Beispiele erläutern die Erfindung weiter:

### Beipiel 1: Klonierung eines 23,3 Kb PCR-Fragments:

Aus menschlicher genomischer DNA wurde durch spezifische Primer ein 23,3 Kb großes PCR-Fragment aus dem β-Globin Gen-Bereich amplifiziert. Hierzu wurde ein kommeriziell erhältlicher Kit (Expand™ 20 Kb^{plus} PCR System Boehringer Mannheim, Katalog Nr. 1681 834) verwendet. Dieser Kit enthält ein spezielles Enzymgemisch aus Taq DNA Polymerase, Pwo DNA Polymerase und einem weiteren hitzestabilen Enzym.

Die für die PCR verwendeten Primer haben folgende Sequenzen:
Forward Primer: 5'CACAAGGGCTACTGGTTGCCGATT3' (SEQ. ID. NO. 2)
Reverse Primer: 5'AGCTTCCCAACGTGATCGCCTT3' (SEQ. ID. NO. 3)

- PCR-Bedingungen:: 250 ng human genomic DNA
400 nmol Primer (Forward; Reverse)
0,5 mmol dNTPs
10 µl 5x Expand™ 20 Kb^{plus} Puffer
0,75 µl Expand™ 20 Kb^{plus} Enzymmix
auf 50 µl mit bidest. Wasser aufgefüllt.

- Zyklusbedingungen:: 2 min. bei 92°C 10 Zyklen mit: 10 sec. bei 92°C
30 sec. bei 62°C
18 min. bei 68°C
- 20 Zyklen mit:: 10 sec. bei 92°C
30 sec. bei 62°C
18 min. bei 68°C;
+ Zyklusverlängerung von 20 sec. für jeden Zyklus 7 min. bei 68°C

Die PCR-Fragmente wurden nach der Amplifiation gelelektrophoretisch analysiert und anschließend mit dem High Pure PCR Template Preparation Kit (Boehringer Mannheim Katalog No. 1796828) aufgereinigt. Anschließend wurden die PCR-Fragmente durch eine Polishingreaktion (Erzeugung glatter Enden) und eine Phosphorylierungsreaktion (Anfügung von 5'Phosphaten, da unphosphorylierte Primer verwendet wurden) modifiziert: Für die Modifizierungsreaktion wurde 1 µg gereinigtes 23,3 Kb PCR-Fragment (entsprechend der Ausbeute einer 50 µl PCR-Reaktion) eingesetzt. Die Polishing- und Phosphorylierungsreaktion erfolgte in einer "one tube" Reaktion wie folgt: Zum PCR-Fragment werden 8 µl 5 x T4 DNA Polymerase Puffer +dNTP, 4 µl 10 x Phosphorylierungspuffer +ATP, 1 U T4 DNA Polymerase (Boehringer Mannheim Katalog No1004786) und 10 U T4 Polynukleotid Kinase (Boehringer Mannheim Katalog No 174645) zugegeben. Der Reaktionsansatz wird mit bidestilliertem Wasser auf 40 µl aufgefüllt und anschließend bei 37°C für 20 min. inkubiert. Die modifizierten PCR-Fragmente werden nach der Reaktion mit dem High Pure PCR Template Preparation Kit (Boehringer Mannheim Katalog No 1796828) gereinigt.

Der Expand Vektor II wurde mit SmaI linearisiert und anschließend mit alkalischer Phosphatase dephosphoryliert. Für die Ligation mit 100 ng des Expand Vektors II wurden 200 ng linearisiertes 23,3 kb Expand PCR Fragment eingesetzt (zweifacher molarer Überschuß). Nach Zugabe von 10 µl 2 x Ligationspuffer^{plus} und 5 U T4 DNA Ligase (Boehringer Mannheim Katalog No 799009) wurde auf 20 µl mit bidest. Wasser aufgefüllt und für 30 min. bei Raumtemperatur (22°C) sowie für mindestens 5 min. bei - 70°C inkubiert. Diese Einfrierreaktion bei - 70°C führt im Vergleich zu nicht eingefrorener Ligationsreaktion zu einer Erhöhung der Anzahl an Bakterienkolonien um das 2 - 4fache.

Für die anschließende Verpackungsreaktion mit dem DNA Packaging Kit wurden 5 µl des Ligationsansatzes zu 25 µl DNA Packaging Extrakt (single tube System, Boehringer Mannheim, Katalog No 1758772) gegeben und für 2 Std. bei Raumtemperatur (22°C) inkübiert. Die Verpackungsreaktion wurde durch Zugabe von 50 µl SM-Puffer (50 mmol Tris/HCL, pH 7,5/22°C; 10 mMol MgSO₄; 100 mM NaCl; 0,01 % Gelatine) sowie 20 µl Chloroform abgestoppt.

Für die Infektion der Bakterienzellen mit dem Phagenüberstand wurde eine E. coli-Magnesiumkultur in 10 mM Magnesiumsulfat hergestellt (Hohn, T. Gene 11, 291-298, 1980). Hierzu wurde der Stamm E. coli DH5 α bis zu einer OD₆₀₀ von 1,0 - in LB-Medium mit Zusatz von Maltose und Magnesiumsulfat (0,2% Maltose; 10mM MgSO₄) angezüchtet. Nach Zentrifugation wurde das Bakterienpellet in einem entsprechendem Volumen an 10 mM MgSO₄ resuspendiert, sodaß eine OD₆₀₀ von 1,0 erhalten wurde. 50 µl dieser E. coli Magnesiumkultur wurden mit 25µl Phagenüberstand (2 Volumen Magnesiumkaltur, 1 Volumen Phagenüberstand) für 30 min. bei Raumtemperatur (22°C) inkubiert. Prinzipiell ist es jedoch auch möglich, bis zu 500 µl der Magnesiumkultur mit bis zu 250 µl Phagenüberstand zu inkubieren, solange das Volumenverhältnis 2:1 beträgt. Nach Zugabe von 100 - 1000 µl LB-Medium und einer Inkubation von 60 min. bei 37°C wurden die infizierten Bakterienzellen auf LB-Planen mit Ampicillin (100 µg/ml) ausplattiert und über Nacht bei 37°C inkubiert.

Bei diesem Experiment wurden auf einer Agarplatte 132 Bakterienkolonien erhalten. Die erhaltenen Klone wurden in LB-Medium mit Ampicillin (100 µg/ml) über Nacht bei 37°C geschüttelt. Die Cosmid-DNA wurde nach Standärdmethoden isoliert (Maniatis et. al. Molecular Cloning. A Laboratoy Manual, Cold Spring Harbour, Laboratory Press. Cold Spring Harbour, NY, 1982) und durch Restriktionsspaltung mit Not I/Bln I analysiert. Von 50 durch Spaltung mit Restriktionsenzymen analysierten Klonen waren 46 positiv (92%), d. h. diese enthielten das zu klonierende PCR-Fragment (23,3 Kb) mit korrekter Größe und zu erwartendem Restriktionsmuster.

### Beispiel 2: Klonierung eines DNA Fragments mit überhängenden Enden:

Ein durch Spaltung mit Eco RI erzeugtes 8576 Basenpaar langes Fragment aus dem Phagen SPP I wurde nach Auftrennung durch Agarosegelelektrophorese und Isolierung aus dem Gel (Konzentration:500 ng in 20 µl) durch Zugabe von 8 µl 5 x T4 DNA Polymerase Puffer und 1 U T4 DNA Polymerase in einem Endvolumen von 40 µl (Auffüllen mit bidest. Wasser) durch Inkubation bei 37°C für 20 min. gepolisht (Erzeugung von glatten Enden). Das DNA Fragment wurde anschließend mit High Pure PCR Template Preparation Kit (Boehringer Mannheim) gereinigt und anschließend in den mit SmaI linearisierten und mit alkalischer Phosphatase dephosphorylierten Expand Vektor I (zweifach molarem Überschuß an Insert gegenüber Vektor) ligiert. Analog zu Beispiel 1 wurden 5 µl (ein Viertel) der Ligationsreaktion zu 25 µl DNA Packaging Extrakt (Single Tube System, Boehringer Mannheim Katalog No 1758 772) gegeben und nach Inkubation bei Raumtemperatur (22°C) für 2 Std. sowie Inkubation mit einer Magnesiumkultur des Stammes E. coli DH5α auf LB Platten mit Ampicillin (150 µg/ml) ausplattiert und über Nacht bei 37°C inkubiert. Die erhaltenen Klone (230) wurden in 10 ml LB Medium mit Ampicillin (150 µg/ml) über Nacht bei 37°C gezuchtet. Die Cosmid-DNA wurde nach Standardmethoden isoliert (Maniatis et. al. 1982) und durch Restriktionsspaltung mit Not I und Not I/Eco RI analysiert. Von 30 untersuchten Klonen enthielten 27 das Insert (90%)) mit korrekter Größe und zu erwartendem Restriktionsmuster.

Die in beiden Beispielen erzielte hohe Klonierungsfrequenz von 90% bzw 92% beweist, daß die erfindungsgemäßen Expandvektoren zur Klonierung besonders großer Fragmente geeignet sind, da Rekombinationsereignisse mit der vergleichsweise großen Vektor-DNA sowie Deletionsereignisse in den klonierten Inserts oder den Vektoren offensichtlich nicht oder nur sehr selten auftreten.

## Patentansprüche

1. Cosmidvektor, dadurch gekennzeichnet, daß der Vektor mindestens 15 kb und maximal 35 kb groß ist

2. Cosmidvektor gemäß Auspruch 1, dadurch gekennzeichnet, daß ein Teil des Vektors identisch mit der vollständigen Sequenz von pHC 79 ist

3. Cosmidvektor gemäß Anspruch 1-2, dadurch gekennzeichnet, daß er als Abstandshalter ein DNA Fragment nicht-eukaryotischen Ursprungs enthält

4. Cosmidvektor gemäß Anspruch 1-3, dadurch gekennzeichnet, daß er außer den COS Sequenzen ein zusätzliches DNA Fragment aus dem Genom des Bakteriophagen Lambda enthält

5. Cosmidvektor gemäß Anspruch 4, dadurch gekennzeichnet,
daß das zusätzliche DNA Fragment ausgewählt wird aus einer Gruppe von Fragmenten bestehend aus dem 8,5 kb großem BamHI/BclI Fragment, dem 16,8 kb großem BamHI Fragment und dem 25,9 kb großen BbrI/BspLu11 Fragment des Bakteriophagen Lambda cI857Sam7

6. Escherichia coli Wirtszelle, enthaltend ein Cosmid gemäß Anspruch 1-5

7. Verwendung von Cosmidvektoren gemäß Anspruch 1-5 zur Klonierung von DNA Fragmenten mit einer Größe zwischen 5 kb und 40 kb

8. Verwendung von Cosmidvektoren gemäß Anspruch 7 zur Klonierung von DNA Fragmenten mit einer Größe zwischen 7 kb und 36 kb

9. Verwendung von Cosmidvektoren gemäß Anspruch 7 zur Klonierung von DNA Fragmenten mit einer Größe, ausgewählt aus einer Gruppe von Bereichen bestehend aus den Intervallen 7 kb - 16,5 kb, 16,5 kb - 25 kb, und 25 kb - 36 kb

10. Verwendung von Cosmidvektoren gemäß Anspruch 7-9, wobei die zu klonierenden DNA Fragmente PCR-Amplifikationsprodukte darstellen

11. Verfahren zur Klonierung von DNA Fragmenten mit einer Größe zwischen 5 kb und 40 kb, wobei Cosmidvektoren gemäß Anspruch 1-5 verwendet werden

12. Verfahren gemäß Anspruch 11,
wobei DNA Fragmente mit einer Größe, ausgewählt aus einer Gruppe von Bereichen bestehendaus den Intervallen 7 kb - 16,5 kb, 16,5 kb - 25 kb, und 25 kb - 36 kb kloniert werden

13. Verfahren gemäß Anspruch 11-12, dadurch gekennzeichnet,
daß PCR Fragmente kloniert werden

14. Verfahren zur Konservierung einer Escherichia coli Suspension in 5-20 mM MgSO₄-Lösung, dadurch gekennzeichnet,
daß die Suspension von einer Temperatur, die niedriger als 30°C, aber höher als 4°C ist, über einen Zeitraum von mindestens 30 Minuten auf -70°C abgekühlt wird

15. Verwendung einer Population von Bakterien, die gemäß Anspruch 14 behandelt wurde, als Wirtsorganismen zur Replikation von *in vitro* verpackter Cosmid-DNA

16. Kit, enthaltend mindestens einen Vektor gemäß Anspruch 1-5

17. Kit gemäß Anspruch 16, zusätzlich enthaltend Lambda DNA packaging Extrakt

18. Kit gemäß Anspruch 17, zusätzlich enthaltend eine Suspension von DH5alpha in MgSO₄-Lösung
